# EUROPEAN PATENT SPECIFICATION

(11) **EP 2 692 320 B1**
(45) Date of publication and mention of the grant of the patent: **07.12.2016**
(21) Application number: 12765329.3
(22) Date of filing: 06.03.2012
(51) Int. Cl.: A61F 13/15, A61F 13/49

(54) **APPARATUS FOR MANUFACTURING DISPOSABLE WEARING ARTICLE**
HERSTELLUNGSVERFAHREN FÜR EINEN EINWEGVERBRAUCHSARTIKEL
APPAREIL DE FABRICATION D'UN ARTICLE VESTIMENTAIRE JETABLE

(30) Priority: 30.03.2011 JP 2011076886
(43) Date of publication of application: 05.02.2014
(73) Proprietor: Unicharm Corporation, Ehime 799-0111 (JP)
(72) Inventor: YAMAMOTO, Hiroki, Kanonji-shi, Kagawa 769-1602 (JP)
(74) Representative: Peter, Julian
(86) International application number: PCT/JP2012/055609
(87) International publication number: WO 2012/132776

(56) References cited:
- EP-A1- 1 563 817
- WO-A1-2010/055853
- JP-A- 8 132 576
- US-A1- 2010 175 807

## Description

### [Technical Field]

The present invention relates to a device for manufacturing a disposable wearing article in which elastic members are mounted at a predetermined position on a web that is a member of the disposable wearing article manufactured in continuity and is conveyed in a machine direction.

### [Background Art]

Conventionally, in the step of manufacturing a disposable wearing article such as a disposable diaper, in order to improve the fitting in the leg hole region and the crotch region of the wearer, a method of arranging thread-like elastic members at the position corresponding to the leg hole region and the crotch region is used widely.

Specifically, according to the manufacturing step, two continuous elastic members are arranged in a waveform such that the elastic members cross at every predetermined interval on a web that is a member of the disposable wearing article. Thus, by arranging the elastic members on the web in a waveform, a disposable wearing article having gathers depending on the shape of the leg hole region and the crotch region can be manufactured in continuity.

Patent Document 1 and Patent Document 2 disclose a device for manufacturing a disposable wearing article in which elastic members are mounted in a waveform on a web that is being conveyed. Specifically, around the arm turning spindle provided at the proximal end of the arm member coupled directly to the motor turning spindle or connected via a speed reducer, the arm member swings in the crossing direction of the web along the widthwise direction of the web that is the direction of crossing the machine direction in which the web is supplied, and the elastic members are delivered from the through hole formed at the tip of the arm member. As a result, the elastic members are arranged in a waveform on the web.

Next, by sandwiching the elastic members and the web by a pair of press rolls provided above and below the web, the elastic members supplied on the web are brought in contact and mounted on the web.

### [Citation List]

### [Patent Literature]

[PTL 1] Japanese Unexamined Patent Application Publication No. 2004-159866 (Pages 6 to 8, Figs. 3 and 4)
[PTL 2] Japanese Unexamined Patent Application Publication No. 2010-115284 (Pages 5 and 6, Figs. 3 and 4)

Further, documents US 2010/175807 A1 and EP 1 563 817 A1 disclose a device for manufacturing a disposable wearing article according to the preamble of claim 1.

### [Summary of Invention]

However, the aforementioned conventional device for manufacturing the disposable wearing article has the following problems. That is, because the arm member turns around the arm turning spindle, the tip on which the through hole is formed draws an arc-shaped trajectory. Furthermore, the arm member is arranged at a position that almost matches the tangent line of a pair of press rolls at a position where the peripheral surface of one of the pair configuring the pair is closest to the peripheral surface of the other one of the pair configuring the same. Therefore, the tip on which the through hole is formed moves farther away from a nip line showing the position where the peripheral surface of one of the press rolls configuring the pair of press rolls is closest to the peripheral surface of the other press roll as the arm member swings.

When the arm member is used in this way, at the time of mounting the elastic members on the web to form a waveform, the arm member must be swung more than the amplitude of the elastic members. Therefore, the swing speed of the arm member must be changed abruptly in the proximity of the swinging end of the arm member, and it is a difficult problem to control the arm member in this way.

Additionally, such a change in the swing speed of the arm member can also become a cause of obstruction in improvement of the operating speed of the manufacturing device.

Thus, the present invention is made in view of this situation, and an object thereof is to provide a device for manufacturing a disposable wearing article that can eliminate the control-related limitations of the arm member and the cause of obstruction in improvement of the operating speed resulting from the conventional arrangement of an arm member.

The present invention is defined in claim 1. It refers to a device for manufacturing a disposable wearing article in which by supplying at least a single continuous web as a member of a continuously manufactured disposable wearing article in a machine direction, and by supplying a continuous elastic member while swinging the elastic member in a crossing direction along a widthwise direction of the web that is the direction crossing the machine direction, the elastic member is mounted in an extended state on the web, the device comprising: a pair of press rolls, having a rotation spindle extending in the crossing direction, configured to press the elastic member and the web while rotating along the machine direction; and a guide arm unit configured to supply the elastic member from the front of the pair of press rolls in the machine direction between the pair of press rolls while swinging the elastic member in the crossing direction, wherein the guide arm unit comprises: a motor, having a motor turning spindle, configured to enable reversal of a turning direction of the motor turning spindle; and an arm member in which a through hole from which the elastic member is inserted is formed, and the arm member is swung, in the crossing direction, around an arm turning spindle turned by the motor, in a viewpoint from above a straight line that passes through the center of a widthwise direction of a lower end of the through hole and is parallel to the arm turning spindle, the center of the widthwise direction of the lower end of the through hole is positioned on one side with a reference of a nip line showing a position where a peripheral surface of one press roll configuring the pair of press rolls is closest to the peripheral surface of the other press roll, at both a central position where the arm member does not swing and at a swinging end where the arm member swings the most and the position of the through hole at the swinging end is closer to the nip line than the position of the through hole at the central position.

### [Brief Description of Drawings]

[Fig. 1] Fig. 1 is a perspective view showing a disposable wearing article according to an embodiment of the present invention.
[Fig. 2] Fig. 2 is a diagram for explaining a part of a method of manufacturing the disposable wearing article according to the embodiment of the present invention.
[Fig. 3] Fig. 3 is a perspective view showing an elastic member mounting device according to the embodiment of the present invention.
[Fig. 4] Fig. 4 is a top view (view on arrow A of Fig. 3) showing the elastic member mounting device according to the embodiment of the present invention.
[Fig. 5] Fig. 5 is an enlarged perspective view of a proximity of a guide arm unit according to the embodiment of the present invention.
[Fig. 6] Fig. 6 is an enlarged side view of a proximity of the guide arm unit according to the embodiment of the present invention.
[Fig 7] Fig 7 is an enlarged front view of a proximity of the guide arm unit.
[Fig. 8] Fig. 8 is an explanatory drawing explaining a positional relationship between a guide arm unit and a nip line according to the embodiment of the present invention.
[Fig. 9] Fig. 9 is an explanatory drawing explaining a positional relationship between the guide arm unit and the nip line according to a first modification of the present invention.
[Fig. 10] Fig. 10 is an enlarged front view of a proximity of a guide arm unit according to a second modification.

### [Description of Embodiments]

Next, an embodiment of a device for manufacturing a disposable wearing article is explained with reference to drawings. In the following description of the drawings, the same or similar reference numerals are used to designate the same or similar parts. It will be appreciated that the drawings are schematically shown and the ratio and the like of each dimension are different from the real ones.

Therefore, a specific dimension should be determined in view of the following description. Moreover, among the drawings, the respective dimensional relations or ratios may differ.

### (1) Configuration of the disposable wearing article

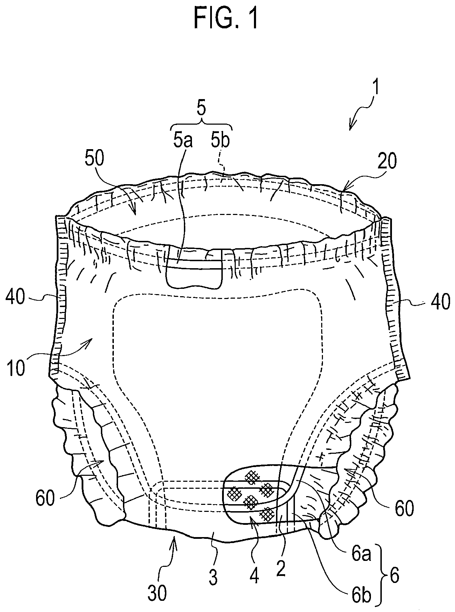

First of all, a configuration of a disposable wearing article according to the present embodiment is explained with reference to drawings. Fig. 1 is a perspective view showing the disposable wearing article according to the present embodiment. In the present embodiment, a disposable wearing article 1 is a disposable diaper for adults.

As shown in Fig. 1, the disposable wearing article 1 is schematically configured from a liquid-permeable topsheet 2 that is in contact with the skin of the wearing subject (hereinafter, the wearer), a backsheet 3 provided on the outer side from the topsheet 2, and an absorber 4 provided between the topsheet 2 and the backsheet 3, which absorbs the bodily waste from the wearer.

Note that a liquid-impermeable water-resistive sheet (not shown in the figure) is provided between the backsheet 3 and the absorber 4. In other words, the absorber 4 is provided between the topsheet 2 and the water-resistive sheet

A nonwoven fabric or an aperture plastic film, for example, is used in the topsheet 2. A nonwoven fabric is used in the backsheet 3. Ground pulp, or a mixture of ground pulp and high absorbent polymer grains, for example, is used in the absorber 4. A plastic film, a nonwoven fabric, or a combined sheet of a plastic film and a nonwoven fabric, for example, is used in the water-resistive sheet.

The disposable wearing article 1 has a front waistline region 10 corresponding to the front waistline of the wearer, a back waistline region 20 corresponding to the back waistline of the wearer, and a crotch region 30 corresponding to the crotch of the wearer.

The front waistline region 10 and the back waistline region 20 are formed as one part by a joining unit 40. Waist gathers 5 made from thread-shaped rubber, for example, having elasticity are provided on the circumference of the front waistline region 10 and the back waistline region 20. The waist gathers 5 are configured from an anterior waist gathers 5a positioned in the front waistline region 10 and a posterior waist gathers 5b positioned in the back waistline region 20. A waistline aperture region 50 is formed between the anterior waist gathers 5a and the posterior waist gathers 5b

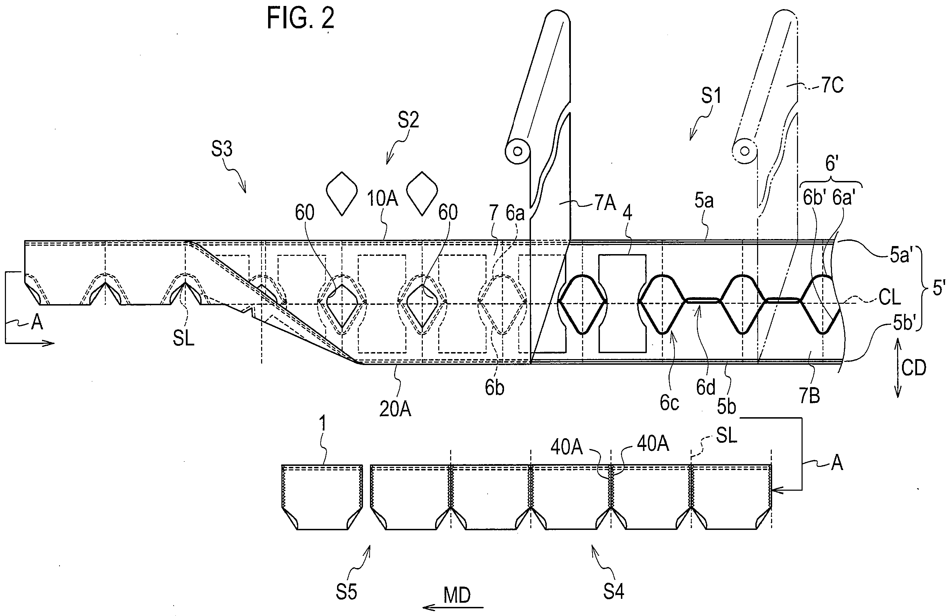

The front waistline region 10 and the back waistline region 20 have elasticity in a machine direction MD of a first web 7A configuring the topsheet 2 and a second web 7B configuring the backsheet 3 (see Fig. 2). For example, the front waistline region 10 and the back waistline region 20 may have elasticity in the machine direction MD due to the provision of waist gathers 5, or may have elasticity in the machine direction MD due to the formation of the front waistline region 10 and the back waistline region 20 by a sheet having elasticity.

The crotch region 30 is provided between the front waistline region 10 and the back waistline region 20. Leg gathers 6 made from thread-shaped or band-shaped rubber having elasticity are provided on both sides of the crotch region 30. The leg gathers 6 are configured from an anterior leg gathers 6a positioned towards the front waistline region 10 and a posterior leg gathers 6b positioned towards the back waistline region 20. A leg-hole aperture region 60 is formed between the anterior leg gathers 6a and the posterior leg gathers 6b, and on both sides of the crotch region 30.

The crotch region 30 has elasticity in the crossing direction CD that crosses the machine direction MD. For example, the crotch region 30 may have elasticity in the crossing direction CD due to the provision of the leg gathers 6, or may have elasticity in the crossing direction CD due to the formation of the crotch region 30 by a sheet having elasticity.

### (2) Method of manufacturing the disposable wearing article

Next, a configuration of the method of manufacturing the disposable wearing article according to the present embodiment is explained with reference to drawings. Fig. 2 is a diagram for explaining a part of the method of manufacturing the disposable wearing article according to the present embodiment.

As shown in Fig. 2, the method of manufacturing the disposable wearing article includes at least a component loading step, a leg hole forming step, a folding step, a joining step, and a cutting step. Note that a step of conveying the liquid-permeable first web 7A configuring the topsheet 2, the liquid-impermeable second web 7B configuring the backsheet 3, and a third web 7C made from the same material as the second web 7B and configuring the backsheet 3 in the machine direction MD using a conveyor (for example, a belt conveyor), which is not shown in the figure, is included between each step.

### (2-1) Component loading step

In the component loading step S1, the components configuring the disposable wearing article 1, such as the elastic members, the third web 7C, the water-resistive sheet (not shown in the figure), and the absorber 4 are loaded on the second web 7B.

Specifically, firstly, an elastic member 5' configuring the waist gathers 5 in an extended state is loaded in a linear state at a position corresponding to the front waistline region 10 and the back waistline region 20 in the second web 7B. That is, an elastic member 5a' configuring an anterior waist gather 5a and an elastic member 5b' configuring a posterior waist gather 5b are loaded at the position corresponding to the front waistline region 10 and the back waistline region 20 in the second web 7B. Thus, waist gathers 5 (anterior waist gathers 5a and posterior waist gathers 5b) are formed at the position corresponding to the front waistline region 10 and the back waistline region 20 in the second web 7B.

Secondly, the third web 7C is loaded on the second web 7B, At this time, an elastic member 6' configuring the leg gathers 6 in an extended state is arranged in a predetermined period while swinging in the crossing direction CD at the position corresponding to the crotch region 30 in the second web 7B and the third web 7C. Thus, the elastic member 6' is sandwiched between the second web 7B and the third web 7C, and forms the leg gathers 6 (anterior leg gathers 6a and posterior leg gathers 6b).

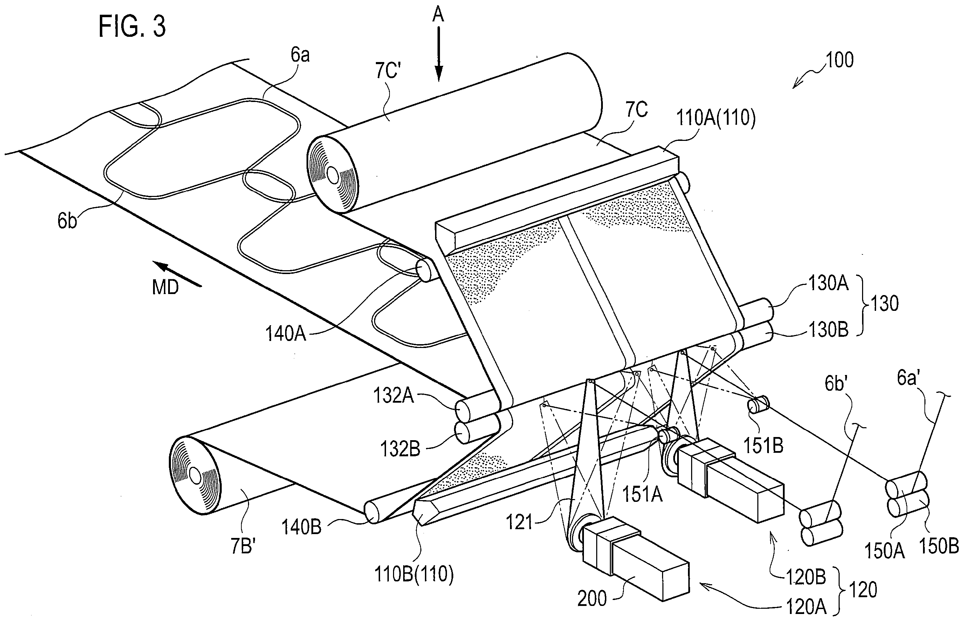

Note that the second web 7B and the third web 7C that sandwich the elastic member 6' are pressed by an upper press roll 130A and a lower press roll 130B (see Fig. 3) described later.

An elastic member 6a' that configures the anterior leg gather 6a and an elastic member 6b' that configures the posterior leg gather 6b form a large annular portion 6c and a small annular portion 6d whose size in the crossing direction CD is smaller than that of the large annular portion 6c.

As described above, after arranging the elastic member 6' in the second web 7B and the third web 7C, the second web 7B and the third web 7C are pressed by the upper press roll 130A and the lower press roll 130B. At this time, unless the position at which the small annular portion 6d is planned to be formed is pressed, the elastic member 6' is not fixed on to the web at the planned position. Because the elastic member 6' is arranged in an extended state, it contracts at the locations where it is not fixed on to the web, and takes an almost linear shape from the predetermined arrangement shape. Thus, the small annular portion 6d is formed.

The same holds true even when an adhesive is not applied at the planned position. Because the elastic member 6' is not fixed on to the web at the planned position where an adhesive is not applied, the small annular portion 6d can be formed in the same way.

Thirdly, a water-resistive sheet (not shown in the figure) and the absorber 4 are loaded on to the second web 7B and the third web 7C while the elastic member 6' is sandwiched in between such that the water-resistive sheet and the absorber 4 are aligned at a fixed interval in the machine direction MD. Note that the water-resistive sheet may be loaded onto the second web 7B and the third web 7C by joining beforehand with the absorber 4, or may be loaded on the second web 7B and the third web 7C separately from the absorber 4.

Fourthly, the first web 7A configuring the topsheet 2 is superposed on to the second web 7B and the third web 7C on which the components configuring the disposable wearing article 1 have been loaded.

Note that the component loading step S1 need not necessarily be performed in the order of first to fourth, and can be changed appropriately.

### (2-2) Leg hole forming step

In the leg hole forming step S2, after the component loading step S1, the leg-hole aperture region 60 (the so-called leg hole) is formed on the second web 7B and the first web 7A (hereinafter, the composite web 7) between which the components are held, by cutting out the inner periphery of the large annular portion 6c.

### (2-3) Folding step

In the folding step S3. after the leg hole forming step S2, the composite web 7 is folded into two through the center of the crossing direction CD with respect to the composite web 7 and along the central line CL toward the machine direction MD. In other words, a side edge 10A of the composite web 7 corresponding to the front waistline region 10, and a side edge 20A of the composite web 7 corresponding to the back waistline region 20 overlap in a matching state.

### (2-4) Joining step

In the joining step S4, after the folding step S3, a predetermined region 40A corresponding to the joining unit 40 of the disposable wearing article 1 is joined by an ultrasonic treatment and a heat treatment. Note that the predetermined region 40A shows both sides of the machine direction MD of the virtual line SL showing the planned cutting position extending in the crossing direction CD.

### (2-5) Cutting step

In the cutting step S5, after the joining step S4, the composite web 7 on to which the predetermined region 40A is joined is cut along the virtual line SL. Thus, the disposable wearing article 1 is formed.

### (3) Configuration of the elastic member mounting device

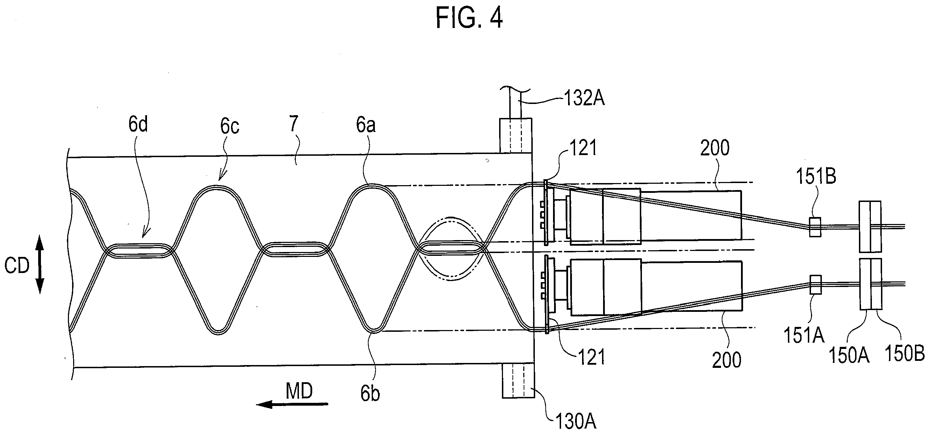

Next, a configuration of an elastic member mounting device 100 used in the aforementioned component loading step S1 is explained with reference to drawings. The elastic member mounting device 100 configures a part of the device for manufacturing the disposable wearing article Fig 3 is a perspective view showing the elastic member mounting device 100 according to the present embodiment. Fig. 4 is a top view (view on arrow A of Fig. 3) showing the elastic member mounting device 100 according to the present embodiment.

As shown in Fig. 3 and Fig. 4, the elastic member mounting device 100 arranges the elastic member 6' configuring the leg gathers 6 between the second web 7B and the third web 7C while swinging the elastic member 6' in the crossing direction CD in a predetermined period. That is, the elastic member mounting device 100 mounts the elastic member 6' in an extended state on the composite web 7 by supplying a plurality of continuous webs (the composite web 7) in the machine direction MD, and by supplying the continuous elastic member 6' while swinging the elastic member 6' in the crossing direction CD along the widthwise direction of the composite web 7 that is the direction of crossing the machine direction MD.

Specifically, the elastic member mounting device 100 forms the leg gathers 6 (anterior leg gather 6a and posterior leg gather 6b) by arranging the elastic member 6' in a waveform between the second web 7B and the third web 7C.

Furthermore, the elastic member mounting device 100 includes a web delivery mechanism (not shown in the figure), a gathers delivery mechanism (not shown in the figure), an adhesive coating mechanism 110, a guide arm unit 120 configured to guide the elastic member 6', and a press roll 130.

### (3-1) Web delivery mechanism

The web delivery mechanism sequentially delivers a web from an original fabric. Specifically, the web delivery mechanism includes an upper web delivery mechanism for sequentially delivering the third web 7C from the third web original fabric 7C', and a lower web delivery mechanism for sequentially delivering the second web 7B from the second web original fabric 7B'.

The upper web delivery mechanism and the lower web delivery mechanism sequentially deliver the second web 7B and the third web 7C, via rollers 140A and 140B that rotate around a rotation spindle (not shown in the figure) running along the crossing direction CD, toward the press roll 130.

### (3-2) Gathers delivery mechanism

The gathers delivery mechanism sequentially delivers the elastic member 6' configuring the leg gathers 6 from an original fabric. Specifically, the gathers delivery mechanism includes an anterior gathers delivery mechanism for sequentially delivering the elastic member 6a' configuring the anterior leg gather 6a from an original fabric (not shown in the figure) and a posterior gathers delivery mechanism for sequentially delivering the elastic member 6b' configuring the posterior leg gather 6b from an original fabric (not shown in the figure).

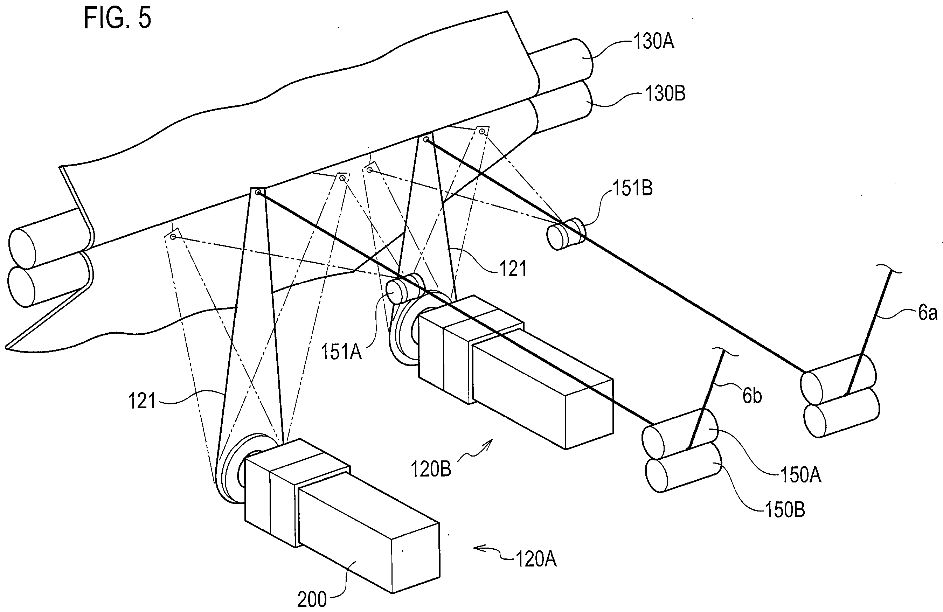

The anterior gathers delivery mechanism and the posterior gathers delivery mechanism sequentially deliver the leg gathers 6 towards the press roll 130 via feed rolls 150A and 150B that rotate around a rotation spindle (not shown in the figure) running along the crossing direction CD, and via splitting rolls 151A and 1551B that split the elastic member 6a' and the elastic member 6b'. Note that in Fig. 3 and Fig. 5, the detailed illustration of splitting of the elastic member 6a' and the elastic member 6b' by the splitting rolls 151A and 151B has been omitted.

### (3-3) Adhesive coating mechanism

The adhesive coating mechanism 110 is a spray-type device for coating an adhesive (for example, a hot-melt adhesive) on the web. Specifically, the adhesive coating mechanism 110 includes an upper adhesive coating mechanism 110A for coating an adhesive on the third web 7C and a lower adhesive coating mechanism 110B for coating an adhesive on the second web 7B.
Note that the upper adhesive coating mechanism 110A is used to coat the adhesive on a surface excluding the central unit of the third web 7C. On the other hand, the lower adhesive coating mechanism 110B is used to coat the adhesive on an entire surface of the second web 7B.

### (3-4) Guide arm unit

The guide arm unit 120 supplies the elastic member 6' from the front of the pair of press rolls 130 in the machine direction MD between the pair of press rolls 130 while swinging the elastic member 6' in the crossing direction CD.
Specifically, the guide arm unit 120 swings the elastic member 6' configuring the leg gathers 6 in the crossing direction CD in a predetermined period, and arranges the elastic member 6' on the web. Specifically, the guide arm unit 120 is configured from a first guide arm unit 120A configured to arrange the elastic member 6a' configuring the anterior leg gather 6a on a web, and a second guide arm unit 120B configured to arrange the elastic member 6b' configuring the posterior leg gather 6b. Note that the first guide arm unit 120A and the second guide arm unit 120B basically have the same configuration. Note that the details of the guide arm unit 120 are described later.

### (3-5) Press roll

The pair of press rolls 130 is configured from an upper press roll 130A that is in contact with the third web 7C, and a lower press roll 130B that is in contact with the second web 7B. The press roll 130 presses the second web 7B and the third web 7C while the elastic member 6' is sandwiched between the second web 7B and the third web 7C.

Specifically, the upper press roll 130A rotates around a rotation spindle 132A extending in the crossing direction CD along the machine direction MD. Similarly, the lower press roll 130B rotates around a rotation spindle 132B extending in the crossing direction CD along the machine direction MD. At the location where the upper press roll 130A and the lower press roll 130B are closest to each other, the elastic member 6' is guided by the guide arm unit 120, and the elastic member 6' is mounted on the second web 7B and the third web 7C.

### (4) Operation of the elastic member mounting device

Next, an operation of the elastic member mounting device 100 according to the present embodiment is explained based on Fig. 3 and Fig. 4.
The third web 7C is delivered from the third web original fabric 7C' by the upper web delivery mechanism, and the direction of movement is changed by the roller 140A. To the surface on which the third web 7C in which the direction of movement is changed faces the second web 7B, an adhesive is coated by the upper adhesive coating mechanism 110A. At that time, the upper adhesive coating mechanism 110A coats the adhesive on a surface excluding the central unit of the third web 7C. The third web 7C on which the adhesive is coated is supplied between the upper press roll 130A and the lower press roll 130B from above.

Similarly, the second web 7B is delivered from the second web original fabric 7B' by the lower web delivery mechanism, and the direction of movement is changed by the roller 140B. To the surface on which the second web 7B in which the direction of movement is changed faces the third web 7C, an adhesive is coated by the lower adhesive coating mechanism 110B. The second web 7B on which the adhesive is coated is supplied between the upper press roll 130A and the lower press roll 130B from below.

The elastic member 6a' is delivered from an original fabric (not shown in the figure) by the anterior gathers delivery mechanism, and the direction of movement is reversed by the feed roll 150A. The elastic member 6a' that is reversed by the feed roll 150A is split by the splitting roll 151A. The split elastic member 6a' moves in the crossing direction CD in a predetermined period by the first guide member 120A. Therefore, the elastic member 6a' is arranged in a curved state between the second web 7B and the third web 7C, and thus forms the anterior leg gather 6a.

Similarly, the elastic member 6b' is delivered from an original fabric (not shown in the figure) by the posterior gathers delivery mechanism, and the direction of movement is reversed by the feed roll 150B. The elastic member 6b' that is reversed by the feed roll 150B is split by the splitting roll 151B. The split elastic member 6b' moves in the crossing direction CD in a predetermined period by the second guide member 120B. Therefore, the elastic member 6b' is arranged in a curved state between the second web 7B and the third web 7C, and thus forms the posterior leg gather 6b.

The elastic member 6a' and the elastic member 6b' are pressed by the upper press roll 130A and the lower press roll 130B while being sandwiched between the second web 7B and the third web 7C on which an adhesive has been coated. Therefore, the elastic member 6a' and the elastic member 6b' are adhered between the second web 7B and the third web 7C while being swung by the guide member 120, and thus, the aforementioned large annular portion 6c is formed. On the other hand, even though the elastic member 6a' and the elastic member 6b' are arranged between the second web 7B and the third web 7C, but because the adhesive is not coated in the central unit of the third web 7C, a restoring force occurs in the elastic member 6a' and the elastic member 6b' and the small annular portion 6d is formed.

### (5) Configuration of the guide arm unit

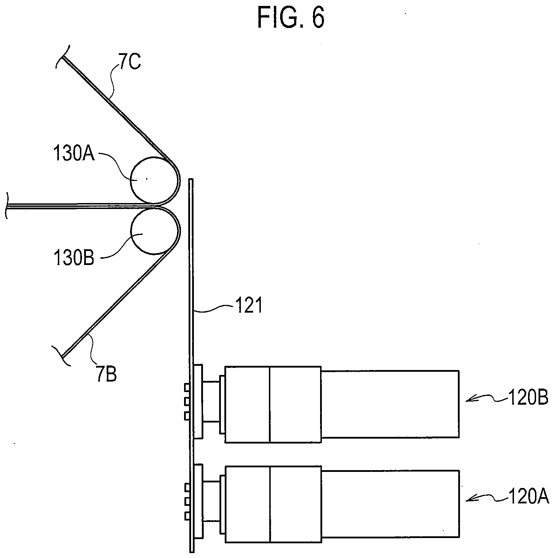

Next, a configuration of the guide arm unit 120, which is a characteristic of the present disclsoure, is explained with reference to Fig. 3 through Fig. 8. The guide arm unit 120 includes a motor 200 and an arm member 121.

The motor 200 is a servo motor including a motor turning spindle 210. The motor 200 rotates the motor turning spindle 210 in a predetermined rotating direction based on the control signal that is input. Furthermore, the turning direction of the motor 200 can be reversed. Thus, the arm member 121 can repeat swinging in a predetermined range.

The arm member 121 guides the elastic member 6' to a predetermined position in the crossing direction CD between the second web 7B and the third web 7C. At the tip of the arm member 121 is formed a through hole 125 through which the elastic member 6' is inserted. Furthermore, at the proximal end of the arm member 121 is formed an arm turning spindle 123 that is turned by motor 200. The arm member 121 is swung around the arm turning spindle 123 in the crossing direction CD In the present embodiment, the arm turning spindle 123 and the motor turning spindle 210 are parallel and overlap each other. That is, the motor 200 and the arm member 121 are arranged such that the arm turning spindle 123 and the motor turning spindle 210 are parallel to each other.

The arm member 121 forms a tapered plate shape from the proximal end to the tip. The arm member 121 is formed by using a metal plate, for example, a stainless steel plate.
Note that the length of the arm member 121 of the first guide arm unit 120A and the length of the arm member 121 of the second guide arm unit 120B may be configured to be different or may be configured to be the same.

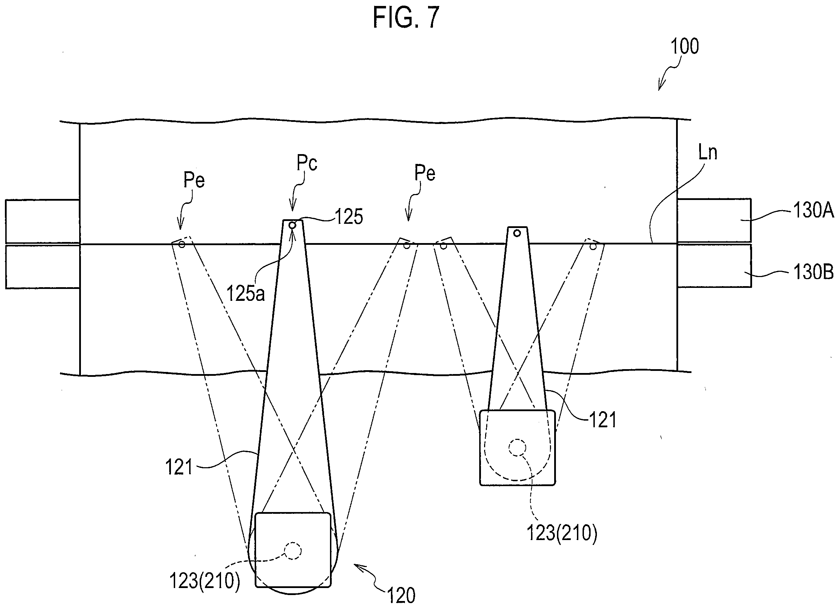

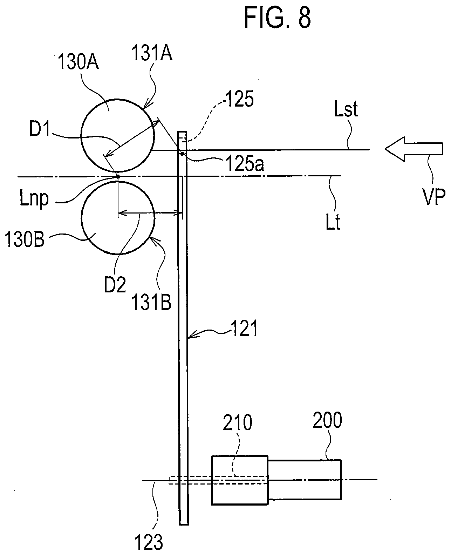

Furthermore, in the present disclosure, the following relationship is established between the arm member 121 and a nip line Ln. Here, as shown in Fig. 7 and Fig. 8, a straight line that passes through the center of the widthwise direction of a lower end 125a of the through hole 125 and is parallel to the arm turning spindle 123 is called a straight line Lst. Note that as shown in Fig. 8, the straight line Lst is parallel to the arm turning spindle 123 in the side surface of the elastic member mounting device 100 (viewpoint from the crossing direction CD), and at the same time, the straight line Lst is parallel to the arm turning spindle 123 even in the viewpoint from the top surface of the elastic member mounting device 100.

Furthermore, the nip line Ln is a line that shows the position where a peripheral surface 131A of one of the press rolls (press roll 130A) configuring the pair of press rolls 130 is closest to a peripheral surface 131B of the other press roll (press roll 130B). That is, the nip line Ln shows the position where the elastic member 6' is mounted (comes in contact) on the second web 7B and the third web 7C.

Fig. 8 shows the positional relationship between the arm member 121 and the nip line Ln as viewed from above the straight line Lst (viewpoint from the arrow VP in Fig. 7). As shown in Fig. 8, the center of the widthwise direction of the lower end 125a of the through hole 125 is positioned on one side with the nip line Ln as the reference, at both a central position Pc where the arm member 121 does not swing and a swinging end Pe where the arm member 121 swings the most.

Specifically, the position of the through hole 125 at the swinging end Pe overlaps the nip line Ln. That is, the position of the through hole 125 at the swinging end Pe is at the same height as the nip line Ln in the viewpoint from the direction in which the straight line Lst is extended. That is, the position of the through hole 125 at the swinging end Pe is closer to the nip line Ln than the position of the through hole 125 at the central position Pc.

Furthermore, as show in Fig. 8, in the side surface view of the elastic member mounting device 100, when the arm member 121 is thus arranged to cross the tangent line Lt of either press roll at the position where the peripheral surface 131A of the press roll 130A is closest to the peripheral surface 131B of the press roll 130B, the swing angle of the arm member 121 is desired to be between 30 degrees and 45 degrees. Note that if the swing angle of the arm member 121 is less than 30 degrees, the length and weight of the arm member 121 increases to acquire the necessary amplitude, and improvement in speed might be restricted due to the high load for the swinging operation, which is not desired. Furthermore, when the swing angle of the arm member 121 exceeds 45 degrees, the position of the straight line Lst moves farther away from the nip line Ln (or the tangent line Lt) by more than what is needed, and at the same time, reaches the nip line Ln while coming in contact with the peripheral surface 131A of the press roll (press roll 130A) due to which the arm member 121 might not be able to draw the accurate trajectory, which is not desired.

### (6) Modifications

Next, modifications of the aforementioned elastic member mounting device 100 are explained. Specifically, modifications concerning the arrangement of the arm member 121 are explained.

### (6-1) First Modification

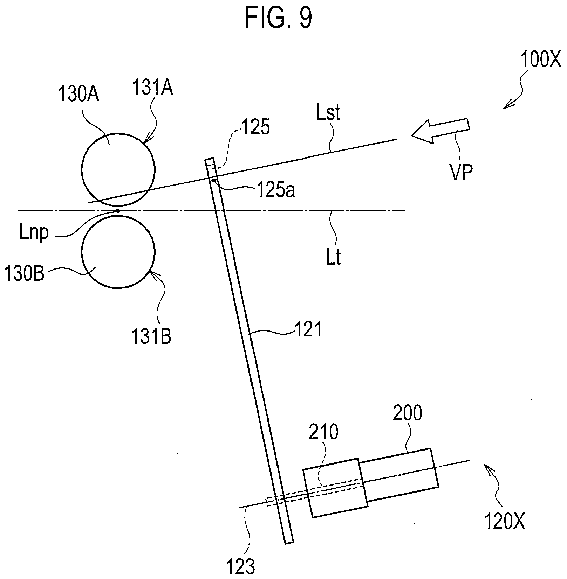

Fig. 9 is a schematic side view of an elastic member mounting device 100X according to the first modification. As shown in Fig. 9, a guide arm unit 120X is arranged at an inclination with respect to the tangent line Lt of either press roll at the position where the peripheral surface 131A of the press roll 130A is closest to the peripheral surface 131 B of the press roll 130B.

Specifically, the arm member 121 is arranged at an inclination with respect to the tangent line Lt. If the inclination angle of the guide arm unit 120 shown in Fig. 8 is 0 degrees, then in view of the effect of the guide arm unit 120, the inclination angle of the arm member 121 is desired to be 45 degrees or less.

Thus, even when the arm member 121 is arranged at an inclination to the tangent line Lt, the positional relationship between the arm member 121 and the nip line Ln as viewed from above the straight line Lst (viewpoint from the arrow VP of Fig. 7) is desired to be such that the center of the widthwise direction of the lower end 125a of the through hole 125 is positioned on one side with the nip line Ln as the reference, at both the central position Pc where the arm member 121 does not swing and the swinging end Pe where the arm member 121 swings the most, as shown in Fig. 8.

### (6-2) Second Modification

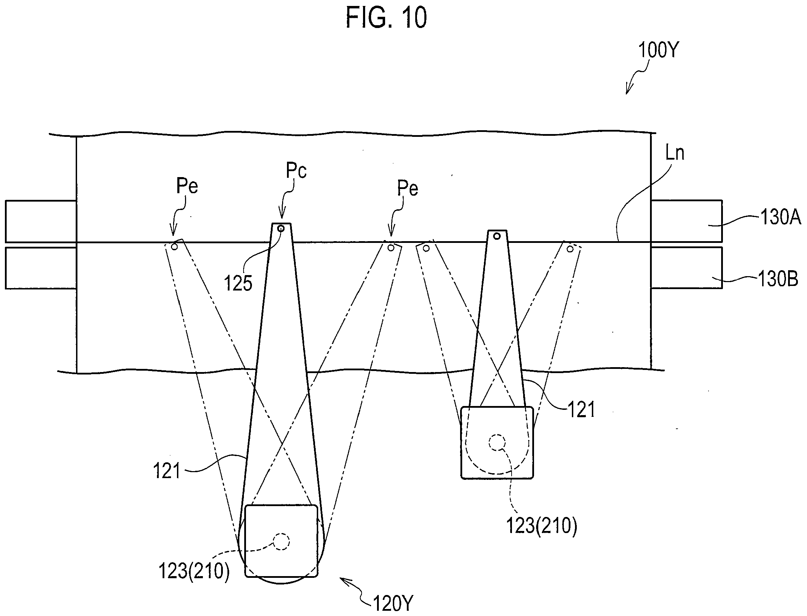

Fig. 10 is a schematic front view of an elastic member mounting device 100Y according to the second modification. As shown in Fig. 10, in a guide arm unit 120Y, the positional relationship between the arm member 121 and the nip line Ln is different from that of the aforementioned elastic member mounting device 100.

Specifically, as viewed from above the straight line Lst (viewpoint from the arrow VP of Fig. 7), the through hole 125 is positioned on one side (upper side) with the nip line Ln as the reference, at the central position Pc where the arm member 121 does not swing. The through hole 125 is positioned on the other side (lower side) with the nip line Ln as the reference, at the swinging end Pe where the arm member 121 swings the most.

That is, the layout and the swing angle of the arm member 121 are determined such that the nip line Ln is formed between the position of the through hole 125 at the central position Pc and the position of the through hole 125 at the swinging end Pe.

According to the elastic member mounting device 100 (100X) explained above, the center of the widthwise direction of the lower end 125a of the through hole 125 formed in the arm member 121 through which the elastic member 6' is inserted is positioned on one side with the nip line Ln as the reference, at both the central position Pc and the swinging end Pe.

Therefore, a distance D2 (see Fig. 8) between the delivery position of the elastic member 6' of the arm member 121 at the swinging end Pe (that is, the position of the through hole 125) and the nip line Ln is smaller than a distance D1 between the delivery position of the elastic member 6' of the arm member 121 at the central position Pc and the nip line Ln. When the elastic member 6' is mounted on the composite web 7 to form a waveform, the arm member 121 must be swung more than the amplitude of the elastic member 6', however, if the delivery position of the elastic member 6' of the arm member 121 at the swinging end Pe comes closer to the nip line Ln in this way, the rate at which the swing speed of the arm member 121 must be changed abruptly in the proximity of the swinging end Pe so as to follow the predetermined arrangement shape of the elastic member 6' is reduced. Specifically, the delivery position of the elastic member 6' can be brought close to the nip line Ln up to the same extent as the so-called direct acting type, without using the arm member 121.

That is, according to the elastic member mounting device 100, the necessity of abruptly changing the swing speed of the arm member 121 is reduced, and the control of the arm member 121 is simplified. Furthermore, because the control of the arm member 121 is simplified, an improvement in the operating speed of the elastic member mounting device 100 can be achieved.

Additionally, according to the elastic member mounting device 100, the arm member 121 is arranged such that the position of the through hole 125 at the swinging end Pe overlaps the nip line Ln. Therefore, the delivery position of the elastic member 6' at the swinging end Pe can be brought closest to the nip line Ln, which further simplifies the control of the arm member 121.

Furthermore, according to the elastic member mounting device 100Y of the second modification, the through hole 125 is positioned on one side with reference to the nip line Ln at the central position Pc, and at the same time, the through hole 125 is positioned on the other side with reference to the nip line Ln at the swinging end Pe. Furthermore, the position of the through hole 125 at the swinging end Pe is closer to the nip line Ln than the position of the through hole 125 at the central position Pc. Therefore, the same action and effect as that as the elastic member mounting device 100 is achieved.

Note that when the through hole 125 is positioned on one side with reference to the nip line Ln at the central position Pc, and at the same time, the through hole 125 is positioned on the other side with reference to the nip line Ln at the swinging end Pe, the position of the through hole 125 at the swinging end Pe need not necessarily be closer to the nip line Ln than the position of the through hole 125 at the central position Pc depending on the layout of the arm member 121. However, even in such a case, when compared to the conventional elastic member mounting device (for example, JPA 2004-159866) in which the arm member 121 is arranged at a position that almost matches the tangent line of the press roll at the position where the peripheral surface of one press roll is closest to the peripheral surface of the other press roll, the difference between the distance D1 and the distance D2 can be reduced, and the aforementioned action and effect can be achieved.

Furthermore, according to the present disclosure, because the swing angle of the arm member 121 is between 30 degrees and 45 degrees, there is no risk that improvement in the speed would be limited due to high load for performing the swing operation, and that the position of the straight line Lst wiN move farther away from the nip line Ln (or the tangent line Lt) by more than what is needed, and reach the nip line Ln while coming in contact with the peripheral surface 131A of the press roll (press roll 130A) due to which the arm member 121 might not be able to draw the accurate trajectory.

### (7) Other embodiments

As mentioned above, although the content of the present invention was disclosed through the embodiments of the present invention, the descriptions and drawings that form a part of this disclosure are not to be considered as limitation to the present invention. From this disclosure, a variety of alternate embodiments, examples, and applicable techniques will become apparent to one skilled in the art.

For example, in the aforementioned embodiment, the arm turning spindle 123 and the motor turning spindle 210 are directly coupled, and the arm turning spindle 123 and the motor turning spindle 210 are arranged parallel (concentrically), however, the arm turning spindle 123 and the motor turning spindle 210 need not necessarily be parallel, and the arm turning spindle 123 and the motor turning spindle 210 may be arranged at an inclination by interposing a bevel gear, for example.

In the aforementioned embodiment, the elastic member 6' is mounted on the composite web 7, specifically, between the second web 7B and the third web 7C, however, for example, the elastic member 6' can also be arranged on a single web. Furthermore, the elastic member 6' may be delivered such that the elastic member 6' is arranged on the top surface of the web, or may be delivered such that the elastic member 6'is arranged on the bottom surface of the web.

According to the characteristic of the present invention, it is possible to provide a device for manufacturing a disposable wearing article that can eliminate the control-related limitations of the arm member and the cause of obstruction in improvement of the operating speed resulting from the conventional arrangement of an arm member.

### [Reference Signs List]

1 ... Disposable wearing article, 2 ... Topsheet, 3 ... Backsheet, 4 ... Absorber, 5 ... Waist gathers, 5a ... Anterior waist gathers, 5b ... Posterior waist gathers, 5', 5a', 5b' ... elastic member, 6 ... Leg gathers, 6a ... Anterior leg gathers, 6b ... Posterior leg gathers, 6c... Large annular portion, 6d ... Small annular portion, 6', 6a', 6b' ... Elastic member, 7 ... Composite web, 7A ...First web, 7B ... Second web, 7B' ... Second web original fabric, 7C ... Third web, 7C'... Third web original fabric, 10 ... Front waistline region, 10A ... Side edge, 20 ... Back leg peripheral region, 20A ... Side edge, 30 ... Crotch region, 40 ... Joining unit, 40A... Predetermined region, 50 ... Waistline aperture region, 60 ... Leg hole aperture region, 100, 100X, 100Y ... Elastic member mounting device, 110 ... Adhesive coating mechanism, 110A ... Upper adhesive coating mechanism, 110B ... Lower adhesive coating mechanism, 120, 120X, 120Y... Guide arm unit, 120A ... First guide arm unit, 120B ... Second guide arm unit, 121 ... Arm member, 123 ... Arm turning spindle, 125 ... Through hole, 125a ... Lower end, 130 ... Press roll, 130A ... Upper press roll, 130B ... Lower press roll, 132A, 132B ... Rotation spindle, 140A, 140B ... Roller, 150A, 150B ... Feed roll, 151 A, 151B ... Splitting roll, 200 ... Motor, 210 ... Motor turning spindle

## Claims

1. A device for manufacturing a disposable wearing article (1) in which by supplying at least a single continuous web as a member of a continuously manufactured disposable wearing article (1) in a machine direction, and by supplying a continuous elastic member while swinging the elastic member in a crossing direction along a widthwise direction of the web that is the direction crossing the machine direction, the elastic member is mounted in an extended state on the web, the device comprising:
a pair of press rolls (130), having a rotation spindle extending in the crossing direction, configured to press the elastic member and the web while rotating along the machine direction; and
a guide arm unit (120) configured to supply the elastic member from the front of the pair of press rolls (130) in the machine direction between the pair of press rolls while swinging the elastic member in the crossing direction, wherein the guide arm unit (120) comprises:
a motor (200), having a motor turning spindle (210), configured to enable reversal of a turning direction of the motor turning spindle (210); and
an arm member (121) in which a through hole (125) from which the elastic member is inserted is formed, and the arm member (121) is swung, in the crossing direction, around an arm turning spindle (123) turned by the motor (200), **characterized in that**
in a viewpoint from above a straight line that passes through the center of a widthwise direction of a lower end of the through hole (125) and is parallel to the arm turning spindle (123), the center of the widthwise direction of the lower end of the through hole (125) is positioned on one side with a reference of a nip line (Ln) showing a position where a peripheral surface of one press roll configuring the pair of press rolls (130) is closest to the peripheral surface of the other press roll, at both a central position where the arm member (121) does not swing and at a swinging end where the arm member (121) swings the most, and
the position of the through hole (125) at the swinging end is closer to the nip line (Ln) than the position of the through hole (125) at the central position.

2. The device for manufacturing a disposable wearing article according to claim 1, wherein according to the viewpoint, the position of the through hole (125) at the swinging end overlaps the nip line (Ln).

3. The device for manufacturing a disposable wearing article according to claim 1, wherein a swing angle of the arm member (121) is between 30 degrees and 45 degrees.

4. The device for manufacturing a disposable wearing article according to claim 1, wherein the motor (200) and the arm member (121) are arranged such that the arm turning spindle (123) and the motor turning spindle (210) are parallel to each other.

## Patentansprüche

1. Vorrichtung zur Herstellung eines wegwerfbaren Trageartikels (1) in welcher durch Zuführen zumindest einer einzelnen endlosen Bahn als ein Teil eines kontinuierlich hergestellten wegwerfbaren Trageartikels (1) in eine Maschinenrichtung und durch Zuführen eines endlosen Elastikelements, während das Elastikelement in eine kreuzende Richtung entlang einer Breitenrichtung der Bahn schwingt, die die Maschinenrichtung kreuzende Richtung ist, das Elastikelement in einem verlängerten Zustand auf der Bahn befestigt ist, die Vorrichtung umfassend:
ein Paar von Presswalzen (130), die eine sich in die kreuzende Richtung erstreckende Rotationsspindel umfassen, ausgebildet während des Rotierens entlang der Maschinenrichtung das Elastikelement und die Bahn zu pressen; und
eine Führungsarmeinheit (120), die ausgebildet ist während des Schwingens des Elastikelements in die kreuzende Richtung das Elastikelement von der Vorderseite des Paares von Presswalzen (130) in die Maschinenrichtung zwischen das Paar von Presswalzen zu führen, wobei die Führungsarmeinheit (120) umfasst:
einen Motor (200) mit einer Motordrehspindel (210), ausgebildet eine Umkehrung einer Drehrichtung der Motordrehspindel (210) zu ermöglichen; und
ein Armelement (121), in dem ein Durchgangsloch (125), von dem das Elastikelement eingebracht ist, geformt ist und wobei das Armelement in die kreuzende Richtung um eine Armdrehspindel (123) gedreht von dem Motor (200) geschwungen ist, **dadurch gekennzeichnet, dass**
in einem Betrachtungspunkt von oberhalb einer gerade Linie, die durch das Zentrum einer Breitenrichtung eines unteren Endes des Durchgangsfochs (125) verläuft und parallel zu der Armdrehspindel (123) ist, ist das Zentrum der Breitenrichtung des unteren Endes des Durchgangsloch (125) auf einer Seite mit einer Referenz einer Klemmlinie (Ln) positioniert, die eine Position zeigt, in der eine periphere Oberfläche einer Presswalze, die das Paar von Presswalzen (130) bildet, am nächsten zu der peripheren Oberfläche der anderen Presswalze ist, an beiden einer Zentralposition an der das Armelement (121) nicht schwingt und an einem schwingendem Ende an dem das Armelement (121) am stärksten schwingt und die Position des Durchgangslochs (125) an dem schwingendem Ende näher zu der Klemmlinie (Ln) ist als die Position des Durchgangslochs (125) an der Zentralposition.

2. Vorrichtung zur Herstellung eines wegwerfbaren Trageartikels nach Anspruch 1, wobei entsprechend des Betrachtungspunkts, die Position des Durchgangslochs (125) an dem schwingenden Ende mit der Klemmlinie (Ln) überlappt.

3. Vorrichtung zur Herstellung eines wegwerfbaren Trageartikels nach Anspruch 1, wobei ein Schwingwinkel des Armelements (121) zwischen 30 Grad und 45 Grad ist.

4. Vorrichtung zur Herstellung eines wegwerfbaren Trageartikels nach Anspruch 1, wobei der Motor (200) und das Armelement (121) so angeordnet sind, dass die Armdrehspindel (123) und die Motordrehspindel (210) parallel zueinander sind.

## Revendications

1. Appareil de fabrication d'un article vestimentaire jetable (1) dans lequel en fournissant au moins une bande continue singulière en tant qu'élément d'un article vestimentaire jetable fabriqué en continu (1) dans un sens machine, et en fournissant un élément élastique continu tout en faisant osciller l'élément élastique dans un sens transversal le long d'un sens de la largeur de la bande qui est la direction croisant le sens machine, l'élément élastique est monté dans un état étendu sur la bande, l'appareil comprenant :
une paire de rouleaux presseurs (130), possédant une broche de rotation s'étendant dans le sens transversal, configurés pour presser l'élément élastique et la bande tout en étant en rotation le long du sens machine ; et
une unité à bras de guidage (120) configurée pour fournir l'élément élastique à partir de l'avant de la paire de rouleaux presseurs (130) dans le sens machine entre la paire de rouleaux presseurs tout en faisant osciller l'élément élastique dans le sens transversal, dans lequel l'unité à bras de guidage (120) comprend :
un moteur (200), possédant une broche de rotation de moteur (210), configurée pour permettre l'inversion d'une direction de rotation de la broche de rotation de moteur (210) ; et
un élément à bras (121) dans lequel un trou débouchant (125) à partir duquel l'élément élastique est inséré est formé, et l'élément à bras (121) est mis en oscillation, dans le sens transversal, autour d'une broche de rotation de bras (123) tournée par le moteur (200), **caractérisé en ce que**
d'un point de sucez partir d'au-dessus d'une ligne droite qui passe à travers le centre d'un sens de la largeur d'une extrémité inférieure du trou débouchant (125) et est parallèle à la broche de rotation de bras (123), le centre du sens de la largeur de l'extrémité inférieure du trou débouchant (125) est positionné sur un côté avec une référence d'une ligne de point de contact (Ln) présentant une position où une surface périphérique d'un rouleau presseur configurant la paire de rouleaux presseurs (130) est plus près de la surface périphérique de l'autre rouleau presseur, à la fois à une position centrale où l'élément à bras (121) n'oscille pas et à une extrémité à oscillation où l'élément à bras (121) oscille le plus, et la position du trou débouchant (125) à l'extrémité à oscillation est plus près de la ligne de point de contact (Ln) que la position du trou débouchant (125) à la position centrale.

2. Appareil de fabrication d'un article vestimentaire jetable selon la revendication 1, dans lequel, selon le point de vue, la position du trou débouchant (125) à l'extrémité à oscillation chevauche la ligne de point de contact (Ln).

3. Appareil de fabrication d'un article vestimentaire jetable selon la revendication 1, dans lequel un angle d'oscillation de l'élément à bras (121) est entre 30 degrés et 45 degrés.

4. Appareil de fabrication d'un article vestimentaire jetable selon la revendication 1, dans lequel le moteur (200) et l'élément à bras (121) sont agencées de telle sorte que la broche de rotation de bras (123) et la broche de rotation de moteur (210) soient parallèles l'une à l'autre.
